# FASCICULE DE BREVET EUROPEEN

(11) **EP 0 868 883 B1**
(45) Date de publication et mention de la délivrance du brevet: **10.03.2004**
(21) Numéro de dépôt: 98400689.0
(22) Date de dépôt: 25.03.1998
(51) Int. Cl.: A61B 18/12

(54) **Catheter pour neurochirurgie**
Katheter für Neurochirurgie
Catheter for neurosurgery

(30) Priorité: 03.04.1997 FR 9704061
(43) Date de publication de la demande: 07.10.1998
(73) Titulaire: Elekta Implants S.A., 06921 Sophia Antipolis (FR)
(72) Inventeur: Lecuyer, Alain, 06130 Grasse (FR)
(74) Mandataire: Geismar, Thierry

(56) Documents cités:
- EP-A- 0 629 382
- DE-A- 3 428 644
- US-A- 5 207 684
- US-A- 5 571 088
- US-A- 5 700 262

## Description

La présente invention concerne un cathéter pour neurochirurgie.

On connaît en neurochirurgie des opérations au cours desquelles on est appelé à perforer une membrane. Il s'agit par exemple de la perforation du plancher du troisième ventricule cérébral dans le traitement de l'hydrocéphalie.

Jusqu'à présent, ces opérations s'effectuent en deux temps. Dans un premier temps, la membrane est ouverte, par exemple à l'aide d'un électrocoagulateur, qui est ensuite retiré. On introduit alors à sa place un cathéter à ballon qui est gonflé afin de dilater l'ouverture.

Il résulte en premier lieu d'une telle procédure une perte de temps, toujours préjudiciable au cours d'une opération Par ailleurs, il peut également se produire un écoulement de fluide céphalo-rachidien lors du retrait de l'électrocoagulateur.

Les documents US-5 571 088 et DE-3 428 644 décrivent respectivement un cathéter pour chirurgie cardio-vasculaire et un cathéter pour le système veineux et artériel d'un patient comprenant un corps tubulaire à l'intérieur duquel est agencé un fil d'alimentation électrique pour une pointe d'électrocoagulation fixée au-delà du ballon. Ces cathéters ne sont cependant pas adaptés pour la neurochirurgie.

La présente invention vise à pallier ces inconvénients.

A cet effet, l'invention a pour objet un cathéter pour neurochirurgie selon la revendication 1 comprenant un corps tubulaire à l'extrémité duquel est monté un ballon de dilatation, ledit corps étant agencé pour être raccordé à des moyens de gonflage du ballon et comportant en outre à son intérieur un fil d'alimentation électrique pour une pointe d'électrocoagulation fixée au delà du ballon.

Ainsi, le corps tubulaire du cathéter de l'invention remplit deux fonctions. D'une part, il sert de conduit pour le fluide de gonflage du ballon. D'autre part il forme une gaine pour le fil d'alimentation de la pointe d'électrocoagulation.

La perforation de la membrane s'effectue alors en une seule opération. Le cathéter étant mis en place avec la pointe d'électrocoagulation au contact de la membrane à perforer, cette pointe est alimentée et réalise donc une ouverture dans la membrane. Le cathéter est alors légèrement enfoncé de manière à engager partiellement le ballon dans l'ouverture. Ce ballon est enfin gonflé pour dilater l'ouverture après quoi le cathéter est retiré.

Selon l'invention, l'extrémité du corps supporte coaxialement un tube métallique à l'extrémité duquel est soudée la pointe d'électrocoagulation et auquel est raccordé électriquement le fil d'alimentation, le ballon étant monté autour du tube métallique dont la paroi est percée pour permettre le gonflement du ballon.

Egalement dans un mode de réalisation particulier, le ballon a une forme allongée, et possède dans sa partie médiane une restriction de diamètre lui évitant de glisser d'un côté ou de l'autre de l'ouverture lors de son gonflement.

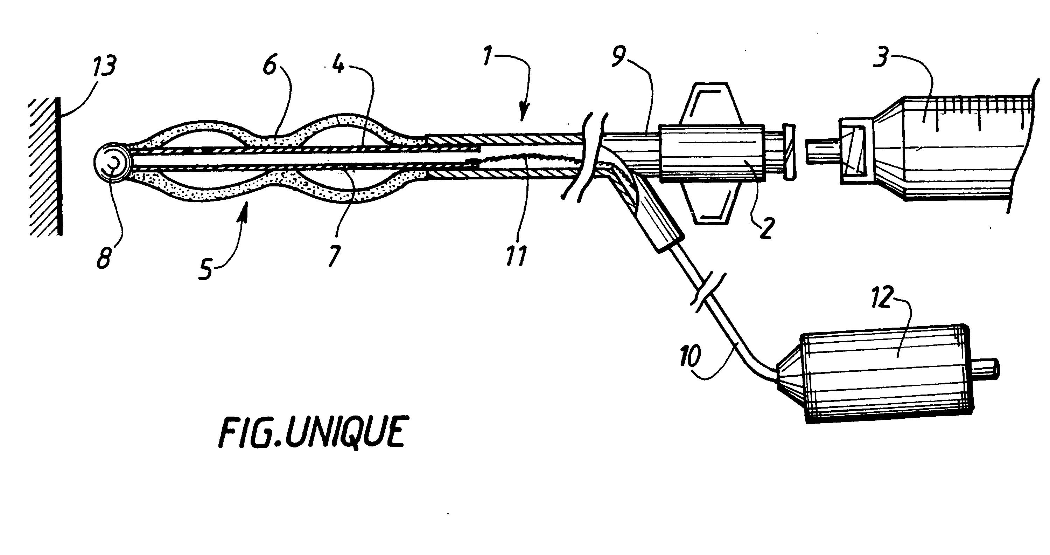

On décrira maintenant, à titre d'exemple non limitatif, un mode de réalisation particulier de l'invention, en référence aux dessins schématiques annexés dans lesquels la figure unique est une vue de côté partiellement en coupe d'un cathéter selon l'invention.

Ce cathéter comprend un corps 1 tubulaire muni à son extrémité extérieure d'un connecteur de gonflage 2. Le connecteur 2 est ici adapté à recevoir l'embout d'une seringue 3.

A son autre extrémité, le corps 1 est raccordé à un tube métallique 4 en acier inoxydable, par exemple par collage, le corps et le tube étant coaxiaux et l'extrémité du tube étant engagée dans celle du corps.

Un ballon en silicone 5 recouvre la surface extérieure du tube 4. Ce ballon 5 est en fait tubulaire et ses deux extrémités sont collées sur le tube de façon étanche. Le ballon 5 comporte en outre dans sa partie médiane une restriction de diamètre 6 dont la fonction sera exposée ci-après.

Par ailleurs, le tube 4 comporte dans sa paroi des trous de gonflage 7 par lesquels le ballon peut être gonflé depuis le connecteur 2, le corps 1, et le tube 4.

Une sphère 8 est soudée à l'extrémité du tube 4 qu'elle obture, au delà du ballon 5.

Le corps 1 forme à proximité du connecteur 2 un raccord 9 dans lequel pénètre de façon étanche un câble 10 électriquement conducteur. Le fil conducteur 11 de ce câble 10 se prolonge dans tout le corps 1 pour être finalement soudé sur le tube 4. L'extrémité extérieure du câble 10 est reliée à un connecteur standard 12 pour sonde électrocoagulante monopolaire. Ainsi, la sphère 8 est reliée électriquement au connecteur 12.

Pour perforer une membrane 13, on amène en premier lieu la sphère 8 à son contact, et on l'alimente à partir du connecteur 12. Une ouverture étant ainsi réalisée dans la membrane, l'extrémité du cathéter y est engagée jusqu'à ce que la membrane se trouve au niveau de la restriction de diamètre 6.

Le ballon 5 est alors gonflé à partir du connecteur 2 pour dilater l'ouverture de la membrane. Du fait de la restriction de diamètre 6, le ballon ne peut glisser d'un côté ou de l'autre de la membrane, et reste donc en place.

Lorsque l'ouverture a atteint une dimension suffisante, le ballon 5 est dégonflé et le cathéter peut être retiré.

## Revendications

1. Cathéter pour neurochirurgie, comprenant un corps tubulaire (1) à l'extrémité duquel est monté un ballon de dilatation (5), ledit corps étant agencé pour être raccordé à des moyens de gonflage (3) du ballon et comportant en outre à son intérieur un fil (11) d'alimentation électrique pour une pointe (8) d'électrocoagulation fixée au delà du ballon,
ledit cathéter étant **caractérisé en ce que** l'extrémité du corps supporte coaxialement un tube métallique (4) à l'extrémité duquel est soudée la pointe d'électrocoagulation et auquel est raccordé électriquement le fil d'alimentation, le ballon étant monté autour du tube métallique dont la paroi est percée pour permettre le gonflement du ballon.

2. Cathéter selon la revendication 1, dans lequel le ballon a une forme allongée, et possède dans sa partie médiane une restriction de diamètre (6).

## Patentansprüche

1. Katheter für Neurochirurgie mit einem röhrenförmigen Körper (1), an dessen Ende ein Ausdehnungsballon (5) angebracht ist, wobei der genannte Körper angeordnet ist, um an Ausdehnungsmittel (3) des Ballons angeschlossen zu werden, und darüber hinaus mit einem elektrischen Versorgungsdraht (11) in seinem Innern für eine außerhalb des Ballons befestigte Elektrokoagulationsspitze (8),
wobei der genannte Katheter **dadurch gekennzeichnet ist, dass** das Ende des Körpers koaxial eine Metallröhre (4) trägt, an deren Ende die Elektrokoagulationsspitze angelötet ist und an der der Versorgungsdraht elektrisch angeschlossen ist, wobei der Ballon um die Metallröhre angebracht ist, deren Wand perforiert ist, um die Ausdehnung des Ballons zu erlauben.

2. Katheter gemäß Anspruch 1, in dem der Ballon eine längliche Form hat und in seinem Mittelteil eine Querschnittsverengung (6) besitzt.

## Claims

1. A catheter for neurosurgery, comprising a tubular body (1) at the end of which an expansion balloon (5) is mounted, the said body being arranged so as to be connected to means (3) of inflating the balloon and also comprising inside it an electrical supply wire (11) for an electrocoagulation tip (8) fixed beyond the balloon,
the said catheter being **characterised in that** the end of the body coaxially supports a metallic tube (4) at the end of which the electrocoagulation tip is welded and to which the supply wire is electrically connected, the balloon being mounted around the metallic tube, whose wall is pierced to allow inflation of the balloon.

2. A catheter according to Claim 1, in which the balloon has an elongate shape and has a restriction in diameter (6) in its middle part.
